(19) Europäisches Patentamt
European Patent Office
Office européen des brevets

(11) **EP 3 460 689 A1**

(12) **EUROPEAN PATENT APPLICATION**
published in accordance with Art. 153(4) EPC

(43) Date of publication:
27.03.2019 Bulletin 2019/13

(51) Int Cl.:
*G06F 19/16* (2011.01)

(21) Application number: **16902299.3**

(22) Date of filing: **16.05.2016**

(86) International application number:
**PCT/JP2016/064443**

(87) International publication number:
**WO 2017/199279 (23.11.2017 Gazette 2017/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventor: **TANIDA, Yoshiaki**
**Kawasaki-shi**
**Kanagawa 211-8588 (JP)**

(74) Representative: **Haseltine Lake LLP**
**Lincoln House, 5th Floor**
**300 High Holborn**
**London WC1V 7JH (GB)**

(54) **METHOD AND DEVICE FOR CALCULATING BINDING FREE ENERGY, AND PROGRAM**

(57) Provided is a method calculating binding free energy, where the method includes adding a distance restraint potential between a binding calculation target molecule and a target molecule, wherein the method is a method for calculating binding free energy between the binding calculation target molecule and the target molecule using a computer, and wherein an anchor point of the target molecule used when the distance restraint potential is added is determined based on a plurality of atoms of the target molecule within a predetermined distance from an anchor point of the binding calculation target molecule, and the anchor point of the target molecule is closer to the anchor point of the binding calculation target molecule than a center of gravity of the target molecule.

FIG. 4

**Description**

FIELD

The embodiments discussed herein relate to a method and device for calculating binding free energy between a target molecule and a binding calculation target molecule, and a program for executing the method.

BACKGROUND

In recent years, simulations have been performed by various computers in order to reduce enormous costs and efforts spent on experimentally searching drug candidate molecules. The search of a drug candidate molecule is to search a compound (ligand) that strongly interacts with a target molecule associated with a target disease (a targeted disease) as a drug candidate. Screening of a compound based on a target molecular steric structure by means of a computer has been actively performed.

Particularly frequently used methods include structure-based drug design (SBDD) (see, for example, NPL 1). The above-mentioned method is a molecule design method based on conformation information of a target molecule or a receptor.

When a drug candidate molecule to be bound to a target molecule is designed using a computer, it is important to quantitatively predict binding activity (binding free energy) of a drug candidate molecule or a fragment of a drug candidate molecule (in the present specification, the drug candidate molecule and the fragment are collectively referred to as a binding calculation target molecule) against a target molecule, in order to efficiently perform feedback to molecular design. In the quantitative prediction of the binding activity, calculation needs to be performed with maintaining a relationship to a standard state in order to directly compare with an experimental value.

Accordingly, in the art, a potential for restraining a distance between a target molecule and a calculation target molecule has been introduced to limit a structural space the molecules can take.

However, in the art, there may be a case where calculation accuracy of binding free energy between the target molecule and the binding calculation target is lowered.

Citation List

Non-Patent Literature

NPL 1: The Process of Structure-Based Drug Design", A.C. Anderson, Chemistry & Biology, 10, 787 (2003)

SUMMARY

The disclosed embodiments aim to solve the above-described various problems existing in the art, and to achieve the following object. Specifically, the present disclosure has an object to provide a method and device for calculating binding free energy where the method and device can improve calculation accuracy of binding free energy between a target molecule and a binding calculation target molecule, and a program for executing the method.

Means for solving the above-described problems are as follows.

The disclosed method for calculating binding free energy includes adding a distance restraint potential between a binding calculation target molecule and a target molecule, wherein the method is a method for calculating binding free energy between the binding calculation target molecule and the target molecule using a computer, and wherein an anchor point of the target molecule used when the distance restraint potential is added is determined based on a plurality of atoms of the target molecule within a predetermined distance from an anchor point of the binding calculation target molecule, and the anchor point of the target molecule is closer to the anchor point of the binding calculation target molecule than a center of gravity of the target molecule.

The disclosed program is a program for causing a computer to execute a method for calculating binding free energy between a binding calculation target molecule and a target molecule. The method includes adding a distance restraint potential between the binding calculation target molecule and the target molecule, and determining an anchor point of the target molecule, which is used when the distance restraint potential is added, based on a plurality of atoms of the target molecule within a predetermined distance from an anchor point of the binding calculation target molecule, and the anchor point of the target molecule is closer to the anchor point of the binding calculation molecule than a center of gravity of the target molecule.

The disclosed device is a device for calculating binding free energy between a binding calculation target molecule and a target molecule. The device includes an addition unit configured to add a distance restraint potential between the binding calculation target molecule and the target molecule, wherein an anchor point of the target molecule used when the distance restraint potential is added is determined based on a plurality of atoms of the target molecule within a predetermined distance from an anchor point of the binding calculation target molecule, and the anchor point of the target molecule is closer to the anchor point of the binding calculation target molecule than a center of gravity of the target molecule.

The disclosed method for calculating binding free energy can improve calculation accuracy of binding free energy between a binding calculation target molecule and a target molecule.

The disclosed program can improve calculation accuracy of binding free energy between a binding calculation target molecule and a target molecule.

The disclosed device for calculating binding free energy can improve calculation accuracy of binding free energy between a binding calculation target molecule and a target molecule.

BRIEF DESCRIPTION OF DRAWINGS

FIG. 1A is a schematic view illustrating one example of a distance restraint potential in the art.
FIG. 1B is a schematic view illustrating one example of a distance restraint potential in the art.
FIG. 2 is a schematic view illustrating one example of a distance restraint potential in the art.
FIG. 3 is a schematic view illustrating one example of a distance restraint potential of the disclosed technology.
FIG. 4 is a conceptual view illustrating one example of the alchemical route calculation method.
FIG. 5A is a schematic view for describing one example of a determination method of an anchor point of a target molecule (part 1).
FIG. 5B is a schematic view for describing one example of the determination method of the anchor point of the target molecule (part 2).
FIG. 5C is a schematic view for describing one example of the determination method of the anchor point of the target molecule (part 3).
FIG. 5D is a schematic view for describing one example of the determination method of the anchor point of the target molecule (part 4).
FIG. 5E is a schematic view for describing one example of the determination method of the anchor point of the target molecule (part 5).
FIG. 6 is a flowchart illustrating one example of the disclosed method for calculating binding free energy.
FIG. 7 is a flowchart illustrating another example of the disclosed method for calculating binding free energy.
FIG. 8 is a view illustrating a structural example of the disclosed device for calculating binding free energy.
FIG. 9 is a view illustrating another structural example of the disclosed device for calculating binding free energy.
FIG. 10 is a view illustrating another structural example of the disclosed device for calculating binding free energy.

DESCRIPTION OF EMBODIMENTS

Drug discovery refers to a process for designing pharmaceutical products. For example, the drug discovery is performed in the following order.

(1) Determination of a target molecule
(2) Searching a lead compound etc.
(3) Examination of physiological effects
(4) Safety/toxicity test

It is important in the search of a lead compound etc. (a lead compound and a compound derived from the lead compound) that interaction between each of numerous drug candidate molecules and a target molecule is highly accurately evaluated.

A process for designing pharmaceutical product using a computer may be referred to as IT drug discovery. The technology of the IT drug discovery can be used for drug discovery in general. Among them, use of the IT drug discovery in a search of a lead compound etc. is effective for reducing a time period for and increasing a probability of developing a new drug.

For example, the disclosed technology can be used for a search of a lead compound etc. that is expected to have high pharmacological activity.

(Method for calculating binding free energy)

The disclosed method for calculating binding free energy is a method for calculating binding free energy between a binding calculation target molecule and a target molecule using a computer.

The inventor of the disclosed technology studied a case of a reduction in calculation accuracy at the time of calculation of binding free energy between a binding calculation target molecule and a target molecule utilizing addition of a distance restraint potential. Then, the cause was considered as follows.

When binding free energy between a binding calculation target molecule and a target molecule is calculated using addition of a distance restraint potential, an anchor point of the binding calculation target molecule and an anchor point of the target molecule are set in order to restrain the binding calculation target molecule and the target molecule.

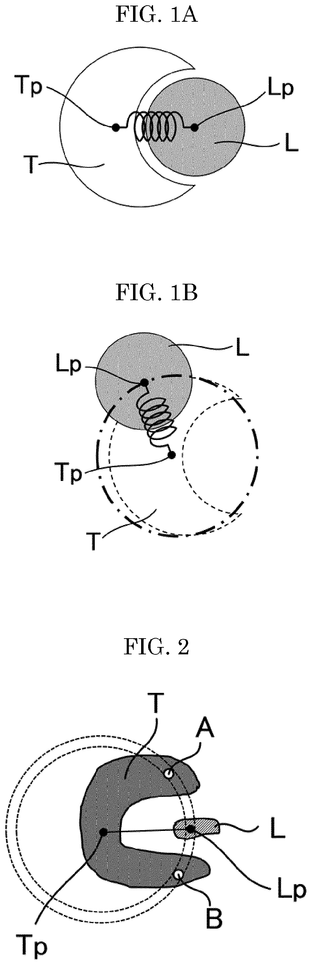

As illustrated in FIG. 1A, a center of gravity of an atom of a binding calculation target molecule L is typically selected as an anchor point Lp of the binding calculation target molecule L. As an anchor point Tp of a target molecule T, a center of gravity of an atom of the target molecule T is typically selected. This is because it is necessary to correlate coordinates of an anchor point with coordinates of the binding calculation target molecule or coordinates of the target molecule, as there is no origin (no fixed point) in a calculation target space.

At the time of calculation of binding free energy, interaction between the binding calculation target molecule L and the target molecule T is eliminated. As a result, the binding calculation target molecule L can move freely on a spherical surface having the anchor point Tp of the binding calculation target molecule L as a center and a distance restrained from the center with the anchor point Tp and the anchor point Lp as a radius as represented by a dot-dash line of FIG. 1B.

With such restraint, however, the binding calculation target molecule L can move on the coordinates on which the target molecule T is originally present. In this case, it is highly possible that the binding calculation target molecule L is trapped by the potential troughs within the target molecule T. As a result, calculated binding free energy may be estimated to be smaller and therefore calculation accuracy decreases.

The inventor of the disclosed technology has made an anchor point of a target molecule closer to an anchor point of a binding calculation target molecule than a center of gravity of the target molecule to thereby make a moving range of the binding calculation target molecule unlikely to overlap with the target molecule. It has been found that, as a result of the above, calculation accuracy of binding free energy to be calculated improves, and the insights as mentioned lead to accomplishment of the disclosed technology.

The concept of the disclosed technology will be described with reference to drawings.

FIG. 2 is a schematic view illustrating a distance restraint potential in the art. Similarly to FIGs. 1A and 1B, in FIG. 2, a center of gravity of a target molecule T is set as an anchor point Tp of the target molecule T. In this case, a moving range of the binding calculation target molecule L is a range indicated with the broken line. Since a distance restraint potential gives a marginal width to a restraining distance, as represented by a restraint potential with a spring, the broken line of FIG. 2 has a width. When the moving range includes therein potential troughs A and B in the target molecule T, the binding calculation target molecule L is trapped by the potential troughs to reduce binding free energy to be calculated. Therefore, calculation accuracy of binding free energy is low.

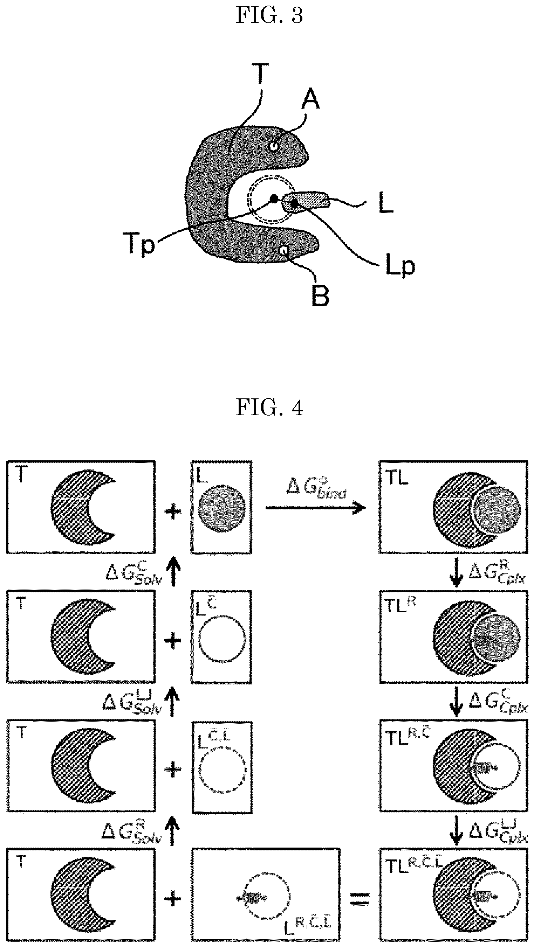

On the other hand, FIG. 3 is a schematic view illustrating a distance restraint potential of the disclosed technology. In FIG. 3, an anchor point Tp of a target molecule T is closer to an anchor point Lp of a binding calculation target molecule L than a center of gravity of the target molecule. Therefore, a moving range of the binding calculation target molecule L is smaller than that in the case of FIG. 2, and the moving range thereof is unlikely to overlap with the target molecule T. As a result, the binding calculation target molecule L is not trapped by the potential troughs A and B in the target molecule T and calculation accuracy of binding free energy improves.

The calculation of the binding free energy is not particularly limited as long as the calculation is a method using a distance restraint potential, and may be appropriately selected depending on the intended purpose. The calculation is more preferably performed according to the alchemical route calculation method. The alchemical route calculation method is also called as alchemical free energy calculation or alchemical transformation, and is a method for calculating binding free energy using a thermodynamic cycle along a virtual (alchemical) path.

For example, the alchemical route calculation method is introduced in Adv Protein Chem Struct Biol. 2011; 85: 27-80.

Examples of the alchemical route calculation method include a calculation method determined by FIG. 4 and the following equation.

$$\Delta G^{\circ}_{bind} = -\left(\Delta G^{C}_{Solv} + \Delta G^{LJ}_{Solv} + \Delta G^{R}_{Solv} + \Delta G^{R}_{Cplx} + \Delta G^{C}_{Cplx} + \Delta G^{LJ}_{Cplx}\right)$$

In FIG. 4, the crescent-shaped object is a target molecule (T) and the circular object is a binding calculation target molecule (L). In the equation above and FIG. 4, C represents electrostatic interaction, LJ represents Van der Waals interaction, Solv represents a solvent, Cplx represents a complex of the target molecule (T) and the binding calculation target molecule (L), and R represent a spring restraint potential.

In the right side of the equation above, the first, second, fourth, fifth, and sixth items can be evaluated, for example, by the Bennett Acceptance Ratio (BAR) method.

Note that, binding free energy of a binding calculating target molecule and a target molecule is typically binding free energy between the binding calculation target molecule and the target molecule that are in a solvent. The solvent is typically water.

Calculation of binding free energy is performed using a computer. The number of the computers used for calculation of the binding free energy may be one, or two or more. For example, calculation of the binding free energy may be performed dividedly by a plurality of computers.

<Distance restraint potential adding step>

The method for calculating binding free energy includes a step including adding a distance restraint potential between the binding calculation target molecule and the target molecule.

An anchor point of the target molecule used when the distance restraint potential is added is determined based on a plurality of atoms within a predetermined distance from an anchor point of the binding calculation target molecule. The plurality of atoms are atoms constituting the target molecule.

The anchor point of the target molecule is closer to an anchor point of the binding calculation target molecule than a center of gravity of the target molecule.

<<Binding calculation target molecule>>

The binding calculation target molecule means a drug candidate molecule, or a fragment for designing a drug candidate molecule.

For example, the fragment is used for fragment-based drug design (FBDD).

<<Target molecule>>

The target molecule is not particularly limited and may be appropriately selected depending on the intended purpose. Examples of the target molecule include protein, ribonucleic acid (RNA), and deoxyribonucleic acid (DNA).

<<Distance restraint potential>>

The distance restraint potential is not particularly limited as long as the distance restraint potential is a potential for restraining a distance between the binding calculation target molecule and the target molecule, and may be appropriately selected depending on the intended purpose. Examples of the distance restraint potential include restraint potentials by springs. The binding force is not particularly limited and may be appropriately selected depending on the intended purpose.

The distance restraint potential is added between the binding calculation target molecule and the target molecule using an anchor point of the binding calculation target molecule and anchor points of the target molecule.

The distance restraint potential added between an anchor point of the binding calculation target molecule and an anchor point of the target molecule is determined, for example, in a manner that a size of fluctuations of the binding calculation target molecule is within a certain range.

The distance restriction between the binding calculation target molecule and the target molecule is performed in order to accurately consider a degree of freedom of translational motions of a molecule contributing the most to binding activity.

Accordingly, it is logical that a center of gravity of the binding calculation target molecule is set as an anchor point of the binding calculation target molecule. For example, a center of gravity of the binding calculation target molecule can be determined by the following equation.

$$\vec{x}_{\mathrm{com}} = \frac{\sum m_i \vec{x}_i}{\sum m_i}$$

In the equation, m represents a mass, and x represents coordinates of an atom constituting the binding calculation target molecule.

Since a hydrogen atom is light, the hydrogen atom hardly affects a position of a center of gravity determined. Accordingly, a center of gravity of the binding calculation target molecule is preferably determined by excluding hydrogen atoms constituting the binding calculation target molecule because the calculation time can be shortened. Atoms excluding hydrogen atoms may be referred to as heavy atoms hereinafter.

<<Anchor point of target molecule>>

An anchor point of the target molecule used when the distance restraint potential is added is determined based on a plurality of atoms within a predetermined distance from an anchor point of the binding calculation target molecule. The plurality of atoms are atoms of the target molecule.

The anchor point of the target molecule is closer to an anchor point of the binding calculation target molecule than a center of gravity of the target molecule.

It is preferable that an anchor point of the target molecule be determined based on a plurality of atoms that are atoms in a binding site of the target molecule within a predetermined distance from an anchor point of the binding calculation target molecule. As a result, the anchor point of the target molecule can be made even closer to an anchor point of the binding calculation target molecule than a center of gravity of the target molecule.

Moreover, the anchor point of the target molecule is preferably a center of gravity of a plurality of atoms that are atoms within a predetermined distance from the anchor point of the binding calculation target molecule and are atoms in a binding site of the target molecule.

Since a hydrogen atom is light, the hydrogen atom has less influence to a position of a center of gravity to be determined. Therefore, a center of gravity of the plurality of atoms of the binding site of the target molecule is preferably determined by excluding hydrogen atoms constituting the binding site of the target molecule because a calculation time can be shortened.

The binding site means a position within the target molecule, at which the target molecule interacts with the binding calculation target molecule. The binding site is also referred to as a ligand binding site.

The plurality of atoms of the binding site selected in the disclosed technology are not limited to certain atoms depending on a target molecule, and may be appropriately selected at the time of calculation considering a structure of the target molecule.

An anchor point of the target molecule is preferably determined using an atom having small fluctuations among atoms in the target molecule.

For example, the atom having small fluctuations is selected by determining the root mean square fluctuation (RMSF) of atoms in the target molecule, and selecting the atom having small RMSF comparing each of the determined RMSF of the atoms.

For example, the root mean square fluctuation (RMSF) was determined on all of the heavy atoms in the target molecule, and the atom having RMSF smaller than the arithmetic mean value of RMSF of all the atoms on which RMSF have been determined is selected as an atom having small fluctuations.

The RMSF of the atom having small fluctuations is preferably 1.0 Å or less.

Examples of the atom having small fluctuations include atoms in a main chain of the target molecule. The main chain means the longest chain in the target molecule. The atoms in the main chain have small fluctuations compared to atoms in side chains.

An anchor point of the target molecule may be a center of gravity of a plurality of atoms having small fluctuations in the target molecule.

Examples of a calculation method of a center of gravity of the plurality of atoms include similar methods for a calculation method of a center of gravity of the binding calculation target molecule.

The predetermined distance is preferably determined by reducing or increasing a space set using an anchor point of the binding calculation target molecule as a reference point. By determining the predetermined distance in the above-mentioned manner, the predetermined distance can be automatically set and a calculation can be automated.

The predetermined distance determined by reducing or increasing a space set using an anchor point of the binding calculation target molecule as a reference point is preferably a distance with which a distance between the anchor point of the binding calculation target molecule and the anchor point of the target molecule determined based on a plurality of atoms of the target molecule is made the shortest. As a result, the moving range of the binding calculation target molecule is unlikely to overlap with the target molecule and calculation accuracy of binding free energy improves even more. Considering that fluctuations of the binding calculation target molecule is typically about 0.3 Å to about 0.5 Å, the distance between the anchor point of the binding calculation target molecule and the anchor point of the target molecule determined based on a plurality of atoms of the target molecule is preferably 2.0 Å or less.

For example, the anchor point can be determined by means of a general computer system (e.g., various network servers, work stations, and personal computers) equipped with a central processing unit (CPU), random access memory (RAM), a hard disk, various peripherals, etc.

One example of a method for making an anchor point of the target molecule used when adding the distance restraint potential closer to an anchor point of the binding calculation target molecule than a center of gravity of the target molecule will be described with reference to drawings.

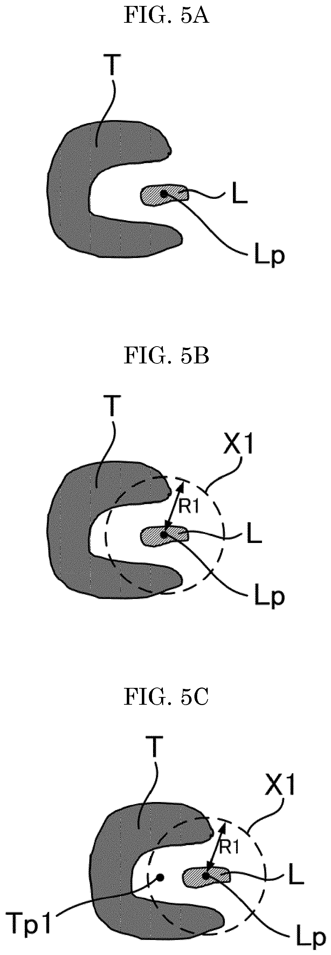

First, a center of gravity of a binding calculation target molecule L is a set as an anchor point Lp of the binding calculation target molecule L (FIG. 5A).

Next, a space X1 having a radius R1 is set using the anchor point Lp of the binding calculation target molecule L as a reference point (origin) (FIG. 5B). In FIG. 5B, the space X1 is a sphere formed with the anchor point Lp as the origin. In the disclosed technology, however, the shape of the space X1 does not need to be a sphere as long as the space X1 is a space determined by a predetermined mathematical formula using the anchor point Lp as a reference point.

Next, a center of gravity of a plurality of atoms of the target molecule T within the space X1 is determined as a candidate anchor point Tp 1 of the target molecule (FIG. 5C).

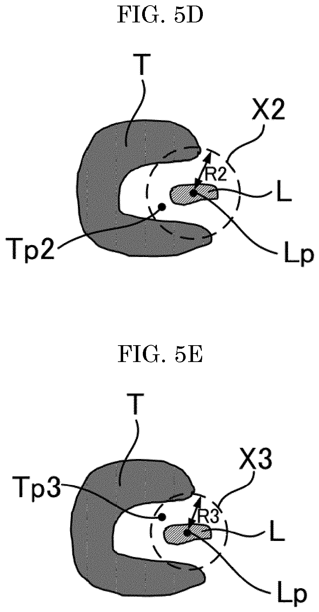

Next, a radius of the space set using the anchor point Lp of the binding calculation target molecule L as a reference point (the origin) is made smaller than the radium R1. Specifically, the space set using the anchor point Lp of the binding calculation target molecule L as a reference point is reduced. Then, a space X2 having a radius R2 that is smaller than the radius R1 is set. Moreover, a center of gravity of a plurality of atoms of the target molecule T within the space X2 is determined as a candidate anchor point Tp2 of the target molecule T (FIG. 5D).

Next, a radius of the space set using the anchor point Lp of the binding calculation target molecule L as a reference point (the origin) is made smaller than the radium R2. Specifically, the space set using the anchor point Lp of the binding calculation target molecule L as a reference point is reduced further. Then, a space X3 having a radius R3 that is smaller than the radius R2 is set. Moreover, a center of gravity of a plurality of atoms of the target molecule T within the space X3 is determined as a candidate anchor point Tp3 of the target molecule T (FIG. 5E).

Furthermore, a process including making a radius the space set using the anchor point Lp of the binding calculation target molecule L as a reference point (the origin) smaller and determining a center of gravity of a plurality of atoms of the target molecule T within a space having the set radius as a candidate anchor point of the target molecule T is repeated.

Among the obtained candidate anchor points, the candidate anchor point from which a distance to the anchor point Lp of the binding calculation target molecule L is the smallest is determined as an anchor point of the target molecule T.

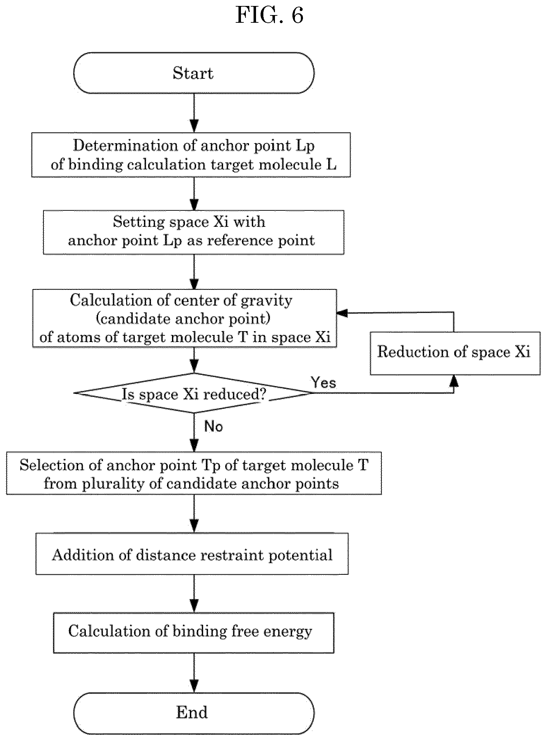

One example of the method for calculating binding free energy will be described with reference to a flowchart (FIG. 6). The method is a method for searching an anchor point of a target molecule by reducing a space set using an anchor point of a binding calculation target molecule as a reference point.

First, an anchor point Lp of a binding calculation target molecule L is determined. For example, the anchor point Lp is a center of gravity of the binding calculation target molecule L.

Next, a space Xi set using the anchor point Lp of the binding calculation target molecule L as a reference point is set. For example, the space Xi is a space having a radius Ri where the space is set using the anchor point Lp as the origin.

Next, a center of gravity of a plurality of atoms of the target molecule T within the space Xi is calculated and determined as a candidate anchor point.

Next, the space Xi is reduced.

Next, a center of gravity of a plurality of atoms of the target molecule T within the reduced space Xi is calculated and determined as a candidate anchor point.

A reduction of the space Xi and calculation of candidate anchor points using the reduced space Xi are repeated several times.

Next, the reduction of the space Xi is terminated, and the candidate anchor point that is the closest to the anchor point Lp of the binding calculation target molecule among the calculated candidate anchor points is selected as an anchor point Tp of the target molecule T.

Next, a distance restraint potential is added between the binding calculation target molecule L and the target molecule T using the anchor point Lp of the binding calculation target molecule L and the anchor point Tp of the target molecule T.

Next, binding free energy between the binding calculation target molecule L and the target molecule T is calculated using the added distance restraint potential.

Then, the example of the calculation of free binding energy is completed.

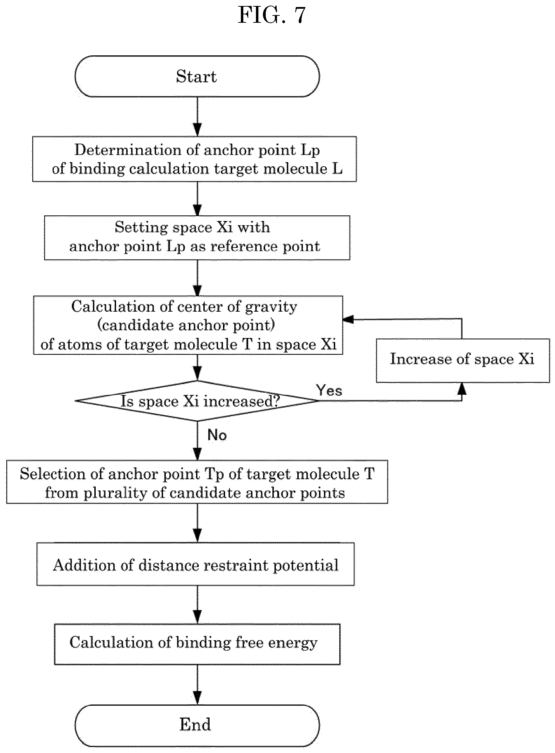

Another example of the method for calculating binding free energy will be described with reference to a flowchart (FIG. 7). The method is a method for searching an anchor point of a target molecule by increasing a space set using an anchor point of a binding calculation target molecule as a reference point.

First, an anchor point Lp of a binding calculation target molecule L is determined. For example, the anchor point Lp is a center of gravity of the binding calculation target molecule L.

Next, a space Xi set using the anchor point Lp of the binding calculation target molecule L as a reference point is set. For example, the space Xi is a space having a radius Ri where the space is set using the anchor point Lp as the origin.

Next, a center of gravity of a plurality of atoms of the target molecule T within the space Xi is calculated and determined as a candidate anchor point.

Next, the space Xi is increased.

Next, a center of gravity of a plurality of atoms of the target molecule T within the increased space Xi is calculated and determined as a candidate anchor point.

Increase of the space Xi and calculation of candidate anchor points using the reduced space Xi are repeated several times.

Next, increase of the space Xi is terminated, and the candidate anchor point that is the closest to the anchor point Lp of the binding calculation target molecule among the calculated candidate anchor points is selected as an anchor point Tp of the target molecule T.

Next, a distance restraint potential is added between the binding calculation target molecule L and the target molecule T using the anchor point Lp of the binding calculation target molecule L and the anchor point Tp of the target molecule T.

Next, binding free energy between the binding calculation target molecule L and the target molecule T is calculated using the added distance restraint potential.

Then, the above-mentioned another example of the calculation of free binding energy is completed.

For example, the method for calculating binding free energy can be performed using the molecular orbital method or the molecular dynamics method.

Examples of molecular orbital calculation according to the molecular orbital method include nonempirical molecular orbital calculation (ab initio molecular orbital calculation), and semiempirical molecular orbital calculation.

Examples of a methodology of the nonempirical molecular orbital calculation include the Hartree-Fock method, and the electron correlation method.

Examples of a methodology of the semiempirical molecular orbital calculation include CNDO, INDO, AM1, and PM3.

Examples of a program of the nonempirical molecular orbital calculation include Gaussian03, GAMESS, ABINIT-MP, and Protein DF.

Examples of a program of the semiempirical molecular orbital calculation include MOPAC.

Examples of a program used for the molecular dynamics method include gromacs (gromacs: Groningen Machine for Chemical Simulations), associated model building with energy refinement (amber), charm, tinker, and lammps.

The method for calculating binding free energy can be performed by using a device for calculating binding free energy described later.


(Program)

The disclosed program is a program for allowing calculation of binding free energy between a binding calculation target molecule and a target molecule to be performed.

With the program, a step including adding a distance restraint potential between the binding calculation target molecule and the target molecule is executed.

In the program, an anchor point of the target molecule used when the distance restraint potential is added is determined based on a plurality of atoms of the target molecule present within the predetermined distance from the anchor point of the binding calculation target molecule and is closer to the anchor point of the binding calculation target molecule than a center of gravity of the target molecule.

The program is configured to execute the method for calculating binding free energy.

The program can be created using any of various programing languages known in the art according to a configuration of a computer system for use, a type or version of an operation system for use.

The program may be recorded on a storage medium, such as an integral hard disk, and an external hard disk, or recorded on a storage medium, such as a compact disc read only memory (CD-ROM), a digital versatile disk read only memory (DVD-ROM), a magneto-optical (MO) disk, and a universal serial bus (USB) memory stick (USB flash drive). In the case where the program is recorded on a storage medium, such as a CD-ROM, a DVD-ROM, an MO disk, and an USB memory stick, the program can be used, as required, directly or by installing a hard disk via a storage medium reader equipped in a computer system. Moreover, the program may be recorded in an external memory region (e.g. another computer) accessible from the computer system via an information and communication network, and the program may be used, as required, by directly from the external memory region or installing into a hard disk from the external memory region via the information and communication network.

(Computer-readable recording medium)

The disclosed computer-readable recording medium has stored therein the disclosed program.

The computer-readable recording medium is not particularly limited and may be appropriately selected depending on the intended purpose. Examples of the computer-readable recording medium include integral hard disks, external hard disks, CD-ROMs, DVD-ROMs, MO disks, and USB memory sticks.

(Device for calculating binding free energy)

The disclosed device for calculating binding free energy is a device for calculating binding free energy between a binding calculation target molecule and a target molecule.

The device for calculating binding free energy includes at least an adding unit configured to perform a step including adding a distance restraint potential between the binding calculation target molecule and the target molecule. The device may further include other units, according to the necessity.

In the device for calculating binding free energy, an anchor point of the target molecule used when the distance restraint potential is added is determined based on a plurality of atoms of the target molecule present within the predetermined distance from the anchor point of the binding calculation target molecule and is closer to the anchor point of the binding calculation target molecule than a center of gravity of the target molecule.

The device for calculating binding free energy is configured to execute the method for calculating binding free energy.

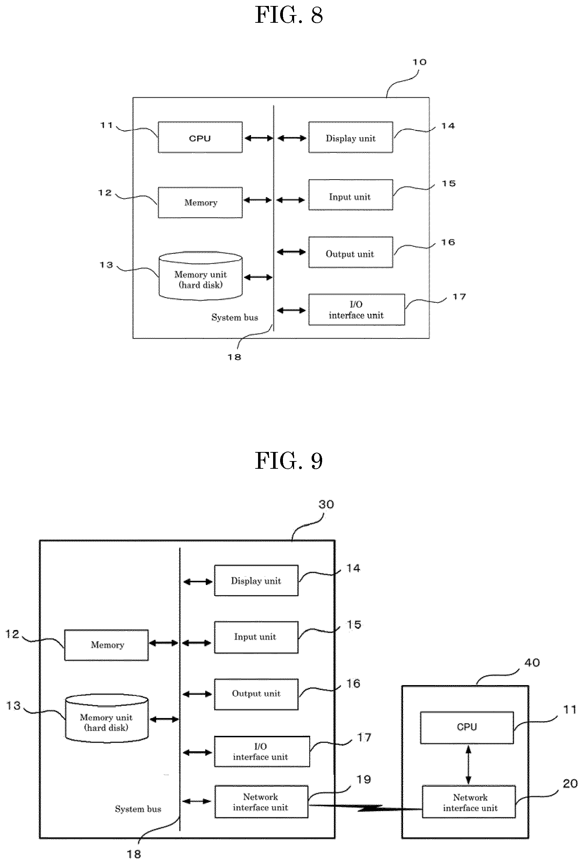

A structural example of the disclosed device for calculating binding free energy is illustrated in FIG. 8.

For example, the device for calculating binding free energy 10 is composed by connecting CPU 11, a memory 12, a memory unit 13, a display unit 14, an input unit 15, an output unit 16, and an I/O interface unit 17 via a system bus 18.

The central processing unit (CPU) 11 is configured to perform calculation (e.g., four arithmetic operation, and relational operation), and control of operations of hardware and software.

The memory 12 is a memory, such as a random access memory (RAM), and a read only memory (ROM). The RAM is configured to store an operation system (OS) and application programs read from the ROM and the memory unit 13, and function as a main memory and work area of the CPU 11.

The memory unit 13 is a device for storing various programs and data. For example, the memory unit 13 is a hard disk. In the memory unit 13, programs to be executed by the CPU 11, data required for executing the programs, and an OS are stored.

The program is stored in the memory unit 13, loaded on the RAM (a main memory) of the memory 12, and executed by the CPU 11.

The display unit 14 is a display device. For example, the display unit is a display device, such as a CRT monitor, and a liquid crystal panel.

The input unit 15 is an input device for various types of data. Examples of the input unit include a key board, and a pointing device (e.g., a mouse).

The output unit 16 is an output device for various types of data. For example, the output unit is a printer.

The I/O interface unit 17 is an interface for connecting to various external devices. For example, the I/O interface unit enables input and output of data of CD-ROMs, DVD-ROMs, MO disks, and USB memory sticks.

Another structural example of the disclosed device for calculating binding free energy is illustrated in FIG. 9.

The structural example of FIG. 9 is a structural example of a cloud-type calculation device, where CPU 11 is independent from a memory unit 13 etc. In the structural example, a computer 30 storing therein the memory unit 13 etc. and a computer 40 storing therein the CPU 11 are coupled with each other via network interface units 19 and 20.

The network interface units 19 and 20 are hardware configured to communicate using the internet.

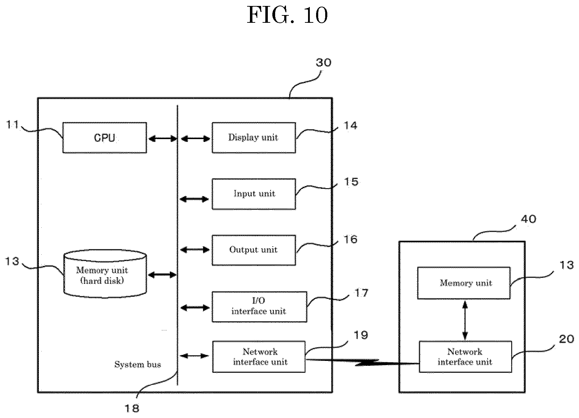

Another structural example of the disclosed device for calculating binding free energy is illustrated in FIG. 10.

The structural example of FIG. 10 is a structural example of a cloud-type calculation device, where a memory unit 13 is independent of CPU 11, etc. In the structural example, CPU 11 etc. are stored via network interface units 19 and 20.

Examples

The disclosed technology will be described hereinafter, but Examples below shall not be construed as to limit the scope of the disclosed technology.

(Example 1)

RNA was used as a target molecule and Theophylline was used as a binding calculation target molecule. The experimental value of binding free energy of a binding structure (conjugate) of RNA and Theophylline is -8.92 kcal/mol (Jenison, R. D.; Gill, S. C.; Pardi, A.; Polisky, B. Science, 1994, 263, 1425-1429).

Binding free energy between RNA and Theophylline was calculated using the disclosed technology according to the flowchart of FIG. 7. As a result, the binding free energy was -8.20 kcal/mol and the result of high calculation accuracy was obtained.

Note that, an anchor point of the binding calculation target molecule was a center of gravity of a heavy atom of the binding calculation target molecule. A candidate anchor point of the target molecule was a center of gravity of a plurality of heavy atoms of the target molecule within a space. In Example 1, the anchor point of the target molecule was a point that has a distance of 1.4 Å from the anchor point of the binding calculation target molecule.

(Comparative Example 1)

Binding free energy was calculated in the same manner as in Example 1, except that the anchor point of the target molecule was changed to a heavy atom of the target molecule. As a result, the calculated binding free energy was -6.30 kcal/mol and the result of low calculation accuracy was obtained.

Reference Signs List

10: device for calculating binding free energy
11: CPU
12: memory
13: memory unit
14: display unit
15: input unit
16: output unit
17: I/O interface unit
18: system bus
19: network interface unit
20: network interface unit
30: computer
40: computer
A: potential trough
B: potential trough
L: binding calculation target molecule
Lp: anchor point
T: target molecule
Tp: anchor point

**Claims**

1. A method for calculating binding free energy, the method comprising:
adding a distance restraint potential between a binding calculation target molecule and a target molecule,
wherein the method is a method for calculating binding free energy between the binding calculation target molecule and the target molecule using a computer, and wherein an anchor point of the target molecule used when the distance restraint potential is added is determined based on a plurality of atoms of the target molecule within a predetermined distance from an anchor point of the binding calculation target molecule, and the anchor point of the target molecule is closer to the anchor point of the binding calculation target molecule than a center of gravity of the target molecule.

**2.** The method according to claim 1,
wherein the anchor point of the target molecule is determined based on a plurality of atoms in a binding site of the target molecule within a predetermined distance from the anchor point of the binding calculation target molecule, and the anchor point of the target molecule is closer to the anchor point of the binding calculation target molecule than a center of gravity of the target molecule.

**3.** The method according to claim 2,
wherein the anchor point of the target molecule is a center of gravity of a plurality of atoms in a binding site of the target molecule within the predetermined distance from the anchor point of the binding calculation target molecule, and the anchor point of the target molecule is closer to the anchor point of the binding calculation target molecule than a center of gravity of the target molecule.

**4.** The method according to any one of claims 1 to 3,
wherein the anchor point of the binding calculation target molecule is a center of gravity of the binding calculation target molecule.

**5.** The method according to any one of claims 1 to 4,
wherein the anchor point of the target molecule is determined using a plurality of atoms having small fluctuations in the target molecule.

**6.** The method according to any one of claims 1 to 5,
wherein the predetermined distance is determined by reducing or increasing a space set using the anchor point of the binding calculation target molecule as a reference point.

**7.** The method according to claim 6,
wherein the predetermined distance determined by reducing or increasing the space set using the anchor point of the binding calculation target molecule as a reference point is a distance with which a distance between the anchor point of the binding calculation target molecule and the anchor point of the target molecule determined based on a plurality of atoms of the target molecule is made the shortest.

**8.** The method according to any one of claims 1 to 7,
wherein the method is performed according to the alchemical route calculation method.

**9.** A program for causing a computer to execute a method for calculating binding free energy between a binding calculation target molecule and a target molecule, the method comprising:
adding a distance restraint potential between the binding calculation target molecule and the target molecule, wherein an anchor point of the target molecule used when the distance restraint potential is added is determined based on a plurality of atoms of the target molecule within a predetermined distance from an anchor point of the binding calculation target molecule, and the anchor point of the target molecule is closer to the anchor point of the binding calculation target molecule than a center of gravity of the target molecule.

**10.** The program according to claim 9,
wherein the anchor point of the target molecule is determined based on a plurality of atoms in a binding site of the target molecule within a predetermined distance from the anchor point of the binding calculation target molecule, and the anchor point of the target molecule is closer to the anchor point of the binding calculation target molecule than a center of gravity of the target molecule.

**11.** The program according to claim 10,
wherein the anchor point of the target molecule is a center of gravity of a plurality of atoms in a binding site of the target molecule within the predetermined distance from the anchor point of the binding calculation target molecule, and the anchor point of the target molecule is closer to the anchor point of the binding calculation target molecule than a center of gravity of the target molecule.

**12.** The program according to any one of claims 9 to 11,
wherein the anchor point of the binding calculation target molecule is a center of gravity of the binding calculation target molecule.

**13.** The program according to any one of claims 9 to 12,
wherein the anchor point of the target molecule is determined using a plurality of atoms having small fluctuations in the target molecule.

**14.** The program according to any one of claims 9 to 13,
wherein the predetermined distance is determined by reducing or increasing a space set using the anchor point of the binding calculation target molecule as a reference point.

**15.** The program according to claim 14,
wherein the predetermined distance determined by reducing or increasing the space set using the anchor point of the binding calculation target molecule as a reference point is a distance with which a distance between the anchor point of the binding calculation target molecule and the anchor point of the target molecule determined based on a plurality of atoms of the target molecule is made the shortest.

**16.** The program according to any one of claims 9 to 15,
wherein the calculation of binding free energy is performed according to the alchemical route calculation method.

**17.** A device for calculating binding free energy between a binding calculation target molecule and a target molecule, the device comprising:
an addition unit configured to add a distance restraint potential between the binding calculation target molecule and the target molecule,
wherein an anchor point of the target molecule used when the distance restraint potential is added is determined based on a plurality of atoms of the target molecule within a predetermined distance from an anchor point of the binding calculation target molecule, and the anchor point of the target molecule is closer to the anchor point of the binding calculation target molecule than a center of gravity of the target molecule.

**18.** The device according to claim 17,
wherein the anchor point of the target molecule is determined based on a plurality of atoms in a binding site of the target molecule within a predetermined distance from the anchor point of the binding calculation target molecule, and the anchor point of the target molecule is closer to the anchor point of the binding calculation target molecule than a center of gravity of the target molecule.

**19.** The device according to claim 18,
wherein the anchor point of the target molecule is a center of gravity of a plurality of atoms in a binding site of the target molecule within the predetermined distance from the anchor point of the binding calculation target molecule, and the anchor point of the target molecule is closer to the anchor point of the binding calculation target molecule than a center of gravity of the target molecule.

**20.** The device according to any one of claims 17 to 19,
wherein the anchor point of the binding calculation target molecule is a center of gravity of the binding calculation target molecule.

**21.** The device according to any one of claims 17 to 20,
wherein the anchor point of the target molecule is determined using a plurality of atoms having small fluctuations in the target molecule.

**22.** The device according to any one of claims 17 to 21,
wherein the predetermined distance is determined by reducing or increasing a space set using the anchor point of the binding calculation target molecule as a reference point.

**23.** The device according to claim 22,
wherein the predetermined distance determined by reducing or increasing the space set using the anchor point of the binding calculation target molecule as a reference point is a distance with which a distance between the anchor point of the binding calculation target molecule and the anchor point of the target molecule determined based on a plurality of atoms of the target molecule is made the shortest.

**24.** The device according to any one of claims 17 to 23,
wherein the calculation of binding free energy is performed according to the alchemical route calculation method.

FIG. 1A

FIG. 1B

FIG. 2

FIG. 3

FIG. 4

FIG. 5A

FIG. 5B

FIG. 5C

FIG. 5D

FIG. 5E

# FIG. 6

```
                    ┌──────────────────────────┐
                    │          Start           │
                    └──────────────────────────┘
                                 │
                                 ▼
              ┌──────────────────────────────────────┐
              │   Determination of anchor point Lp    │
              │ of binding calculation target molecule L │
              └──────────────────────────────────────┘
                                 │
                                 ▼
              ┌──────────────────────────────────────┐
              │         Setting space Xi with         │
              │   anchor point Lp as reference point   │
              └──────────────────────────────────────┘
                                 │
                                 ▼
          ┌──────────────────────────────────────────┐
          │      Calculation of center of gravity      │           ┌──────────────────────┐
          │        (candidate anchor point)            │◄──────────│ Reduction of space Xi │
          │ of atoms of target molecule T in space Xi  │           └──────────────────────┘
          └──────────────────────────────────────────┘                         ▲
                                 │                                              │
                                 ▼                         Yes                  │
                      ◇ Is space Xi reduced? ◇───────────────────────────────────┘
                                 │
                                 │ No
                                 ▼
        ┌──────────────────────────────────────────────┐
        │ Selection of anchor point Tp of target molecule T │
        │   from plurality of candidate anchor points     │
        └──────────────────────────────────────────────┘
                                 │
                                 ▼
              ┌──────────────────────────────────────┐
              │   Addition of distance restraint potential │
              └──────────────────────────────────────┘
                                 │
                                 ▼
              ┌──────────────────────────────────────┐
              │    Calculation of binding free energy   │
              └──────────────────────────────────────┘
                                 │
                                 ▼
                    ┌──────────────────────────┐
                    │           End            │
                    └──────────────────────────┘
```

FIG. 7

```
                    ┌─────────────────────┐
                    │        Start        │
                    └─────────────────────┘
                               │
                               ▼
          ┌──────────────────────────────────────────┐
          │        Determination of anchor point Lp   │
          │   of binding calculation target molecule L │
          └──────────────────────────────────────────┘
                               │
                               ▼
          ┌──────────────────────────────────────────┐
          │           Setting space Xi with           │
          │    anchor point Lp as reference point     │
          └──────────────────────────────────────────┘
                               │
                               ▼
     ┌────────────────────────────────────────┐
     │      Calculation of center of gravity    │        ┌────────────────────────┐
     │         (candidate anchor point)         │◄───────│   Increase of space Xi  │
     │ of atoms of target molecule T in space Xi │        └────────────────────────┘
     └────────────────────────────────────────┘                      ▲
                               │                                      │
                               ▼                         Yes          │
                    ◄─ Is space Xi increased? ───────────────────────┘
                               │
                               │ No
                               ▼
     ┌────────────────────────────────────────────┐
     │ Selection of anchor point Tp of target molecule T │
     │    from plurality of candidate anchor points │
     └────────────────────────────────────────────┘
                               │
                               ▼
          ┌──────────────────────────────────────────┐
          │    Addition of distance restraint potential │
          └──────────────────────────────────────────┘
                               │
                               ▼
          ┌──────────────────────────────────────────┐
          │      Calculation of binding free energy   │
          └──────────────────────────────────────────┘
                               │
                               ▼
                    ┌─────────────────────┐
                    │         End         │
                    └─────────────────────┘
```

FIG. 8

10

11 CPU ↔ ↔ Display unit 14

12 Memory ↔ ↔ Input unit 15

↔ Output unit 16

13 Memory unit (hard disk) ↔ ↔ I/O interface unit 17

System bus

18

FIG. 9

30

↔ Display unit 14

↔ Input unit 15

12 Memory ↔ ↔ Output unit 16

↔ I/O interface unit 17

13 Memory unit (hard disk) ↔

40

CPU 11

19 Network interface unit

↔ Network interface unit 20

System bus

18

FIG. 10

## INTERNATIONAL SEARCH REPORT

| | International application No. |
|---|---|
| | PCT/JP2016/064443 |

**A. CLASSIFICATION OF SUBJECT MATTER**
*G06F19/16(2011.01)i*

According to International Patent Classification (IPC) or to both national classification and IPC

**B. FIELDS SEARCHED**

Minimum documentation searched (classification system followed by classification symbols)
G06F19/16

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
CiNii

**C. DOCUMENTS CONSIDERED TO BE RELEVANT**

| Category* | Citation of document, with indication, where appropriate, of the relevant passages | Relevant to claim No. |
|---|---|---|
| X | JP 2014-186468 A (Fujitsu Ltd.), 02 October 2014 (02.10.2014), paragraphs [0015] to [0032] | 1-4,6-7, 9-12,14-15, 17-20,22-23 |
| Y | & US 2014/0288899 A1 paragraphs [0052] to [0083] & EP 2782033 A1 | 5,8,13,16, 21,24 |
| Y | Yoshiaki TANIDA, "Binding Free Energy Calculation of Small Molecules to Rna with Multiple Binding Poses", [online], J. Comput. Chem. Jpn., vol.14, no.3, 30 September 2015 (30.09.2015), pages 80 to 82, ISSN 1347-3824, DOI 10.2477/jccj.2015-0029, [retrieval date 27 May 2016 (27.05.2016)], Internet, <URL: https://www.jstage.jst.go.jp/article/jccj/14/3/ 14_2015-0029/_pdf> | 5,8,13,16, 21,24 |

| ☒ Further documents are listed in the continuation of Box C. | ☐ See patent family annex. |
|---|---|

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 May 2016 (27.05.16) | 07 June 2016 (07.06.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

21

**INTERNATIONAL SEARCH REPORT**

| International application No. |
|---|
| PCT/JP2016/064443 |

C (Continuation). DOCUMENTS CONSIDERED TO BE RELEVANT

| Category* | Citation of document, with indication, where appropriate, of the relevant passages | Relevant to claim No. |
|---|---|---|
| A | Yoshiaki TANIDA et al., "Binding Free Energy Calculations for Theophylline/Caffeine to RNA Aptamer", J. Comput. Chem. Jpn., vol.13, no.3, 30 September 2014 (30.09.2014), pages 193 to 195, ISSN 1347-1767, DOI 10.2477/jccj.2014-0029 | 1-24 |
| A | US 2011/0130968 A1 (CLARK, Matthew), 02 June 2011 (02.06.2011), entire text (Family: none) | 1-24 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **A.C. ANDERSON.** The Process of Structure-Based Drug Design. *Chemistry & Biology,* 2003, vol. 10, 787
- the alchemical route calculation method is introduced. *Adv Protein Chem Struct Biol.,* 2011, vol. 85, 27-80
- **JENISON, R. D. ; GILL, S. C. ; PARDI, A. ; POLISKY, B.** *Science,* 1994, vol. 263, 1425-1429